# EUROPEAN PATENT SPECIFICATION

(11) **EP 3 019 459 B1**
(45) Date of publication and mention of the grant of the patent: **22.08.2018**
(21) Application number: 14736807.0
(22) Date of filing: 08.07.2014
(51) Int. Cl.: C07C 29/14

(54) **SELECTIVE TRANSFER HYDROGENATION OF RETINAL**
SELEKTIVE TRANSFERHYDRIERUNG VON RETINAL
HYDROGÉNATION DE TRANSFERT SÉLECTIF DE RÉTINAL

(30) Priority: 08.07.2013 EP 13175593
(43) Date of publication of application: 18.05.2016
(60) Divisional application: 17184835.1
(73) Proprietor: DSM IP Assets B.V., 6411 TE Heerlen (NL)
(72) Inventor: BONRATH, Werner, CH-4002 Basel (CH); MEDLOCK, Jonathan Alan, CH-4002 Basel (CH); WÜSTENBERG, Bettina, CH-4002 Basel (CH); SCHÜTZ, Jan, CH-4002 Basel (CH); NETSCHER, Thomas, CH-4002 Basel (CH)
(74) Representative: Kurt, Manfred
(86) International application number: PCT/EP2014/064566
(87) International publication number: WO 2015/004117

(56) References cited:
- US-A- 4 906 795
- E. FACHE ET AL.: "Selective hydrogenation of alpha,beta-unsaturated aldehydes catalyzed by supported aqueous-phase catalysts and supported homogeneous catalysts", JOURNAL OF MOLECULAR CATALYSIS, vol. 79, 1993, pages 117-131, XP002719723,
- XIAOFENG WU ET AL: "A Versatile Iridium Catalyst for Aldehyde Reduction in Water", CHEMSUSCHEM, WILEY - VCH VERLAG GMBH & CO. KGAA, DE, IT, vol. 1, no. 1-2, 22 February 2008 (2008-02-22), pages 71-74, XP008164843, ISSN: 1864-5631, DOI: 10.1002/CSSC.200700086 [retrieved on 2008-01-02]

## Description

The present invention relates to a selective transfer hydrogenation of retinal to retinol.

Retinal is also called retinaldehyde or vitamin A aldehyde.

Retinol (also vitamin A₁) is one form of vitamin A. It is a diterpenoid and an alcohol. It is convertible to other forms of vitamin A, and the retinyl ester derivative of the alcohol serves as the storage form of the vitamin in animals.

Retinal (IUPAC:3,7-dimethyl-9-(2,6,6-trimethylcyclohexen-1-yl)nona-2,4,6,8-tetraenal) is the compound of formula (I)

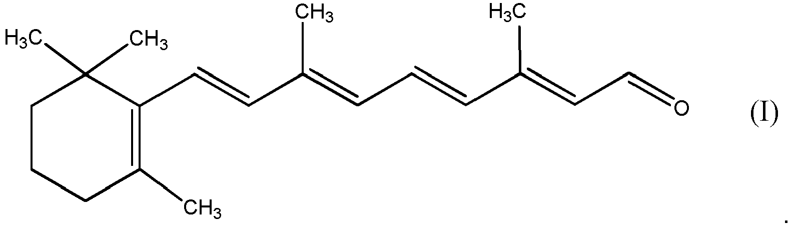

Retinol (IUPAC: 3,7-dimethyl-9-(2,6,6-trimethylcyclohex-1-enyl)nona-2,4,6,8-tetraen-1-ol) is the compound of formula (II)

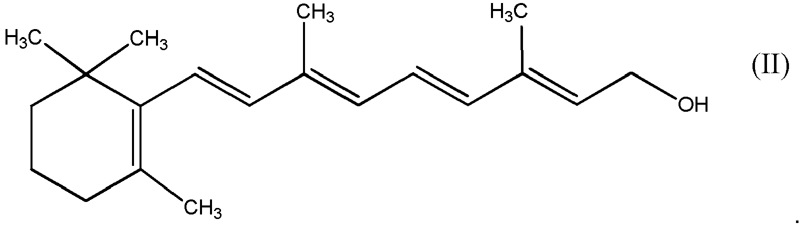

Retinol is an important precursor of vitamin A acetate (or retinyl acetate), Retinyl acetate (retinol acetate, vitamin A acetate) is a stable form of vitamin A which is the acetate ester of retinol. It has potential antineoplastic and chemopreventive activities.

E.Fache et al. in Journal of Molecular Catalysis, Vol. 79, 1993, p.177-131 discloses selective hydrogenations of different α,β-unsaturated aldehydes into allylic alcohols with immobilized homogeneous catalysts.

Retinal as well as retinol can have various isomeric configurations. In the context of the present invention the formula (I) as well as formula (II) are to be understood in the way that they cover all these configurations. Most preferred configuration is (all-E)-retinal/retinol. Other isomers can be converted into (all-E), when needed.

The goal of the present invention was to find an improved way to provide retinol (compound of formula (II)), 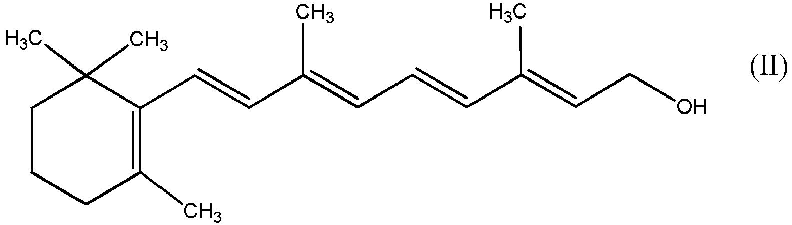 which is the selectively hydrogenated product of retinal.

Surprisingly we found that by using specific kinds of catalysts and reduction agents it is possible to hydrogenate retinal selectively by a transfer hydrogenation reaction whereas the selectivity, the conversion as well as the yield of the hydrogenation are excellent.

The present invention relates to a transfer hydrogenation (TH) of a compound of formula (I) 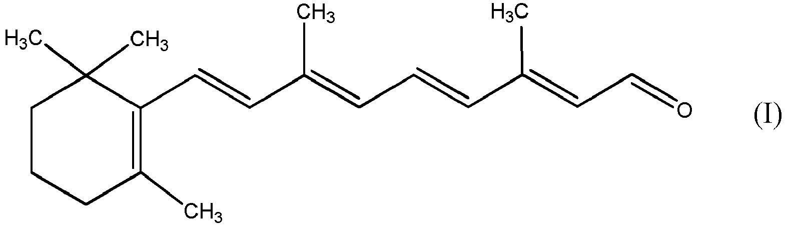 to a compound of formula (II) 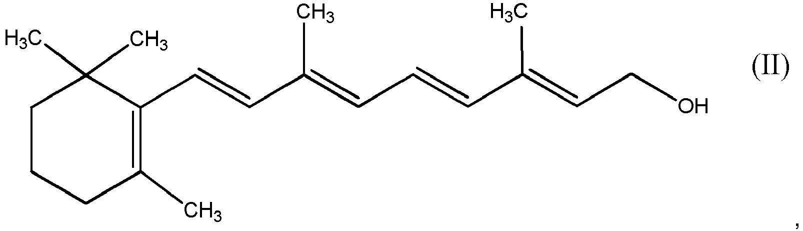 characterized in that the hydrogenation is carried out in the presence of
a) an amino-amide-transition-metal catalyst of formula (III)

   MX[Z-(-H)][Y] (III)

   wherein
   - M: is chosen from the group consisting of Ir, Rh and Ru, and
   - X: signifies H or a halogen atom, and
   - Y: is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
   - Z: signifies a group of formula (IV) or (V) 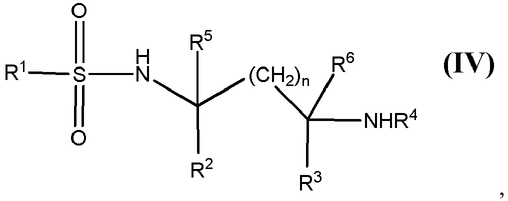 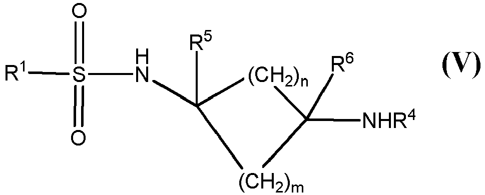
   wherein
   R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
   R² and R³ each independently signify hydrogen, C₁-C₂-alkyl, C₄-C₇-cycloalkyl, or aryl which may be substituted, and
   R⁴ signifies hydrogen or C₁-C₂-alkyl, and
   R⁵ signifies hydrogen or C₁-C₂-alkyl, and
   R⁶ signifies hydrogen or C₁-C₂-alkyl, and
   n signifies 0,1,2 or 3, and
   m signifies 0,1,2 or 3, and
   with the proviso that in formula (V) the sum of n and m is 3 or 4,and
b) a H₂ donor,
   and wherein the hydrogenation is carried out by using at least one solvent which also serves as hydrogen donor.

A preferred transfer hydrogenation (TH¹) is (TH), characterized in that the hydrogenation is carried out in the absence of water.
This (*"in the absence of water"*) means that water is not added intentionally to the reaction solution. The water content is usually below 3 weight-% (wt-%), preferably less than 2 wt-%, based on the total weight of the reaction solution.

The selectivity, the yield and the conversion of the transfer hydrogenation according to the present invention are excellent.

The monosulphonylated diamine is present in the complex as a monoanion and is accordingly denoted in formula III as "Z-(-H)".

By the term "amino-amide transition metal catalyst" it is meant that the transition metal is complexed to Z via the amine and the amide of Z.

Transfer hydrogenation is the addition of hydrogen to a compound (molecule) from a source other than gaseous H₂.
The use of H₂ gas has some issues with the handling of the explosive gas in regard to safety and in regard to the apparatus needed.

Instead of H₂ gas a H₂ donor system is used for transfer hydrogenation. Any H₂ donor system known from the prior art can be used.

The H₂ donor (= reduction agent), which is used in the process according to the present invention, can be any commonly known H₂ donor in the field of transfer hydrogenation.

As the hydrogen donor and at the same time the solvent there can be used especially alcohols, preferably secondary alcohols, e.g. isopropanol.

Therefore a further embodiment of the present invention is a transfer hydrogenation (TH⁴), which is (TH), or (TH¹), wherein the hydrogenation is carried out by using isopropanol as hydrogen donor and solvent.

In the scope of the present invention the term "substituted with one or more fluorine atoms", otherwise expressed as "mono- or multiply-fluorinated", means having at least one fluorine substituent to as many fluorines as the alkyl group so modified is capable of accepting; however one to five fluorines is preferred.

In the scope of the present invention the term "alkyl" embraces straight-chain or branched alkyl groups with 1 to 6 carbon atoms, preferably 1 to 4 carbon atoms. Methyl, ethyl, propyl, isopropyl, n-butyl, isobutyl and tert.-butyl, are examples such alkyl groups. Trifluromethyl, 2,2,2-trifluoroethyl and pentafluoroethyl are examples of mono- or multiply-fluorinated alkyl groups.

The term "alkenyl" embraces straight-chain or branched alkenyl groups with 2 to 8 carbon atoms, preferably 2 to 6 carbon atoms, e.g. allyl, 2-butenyl and 3-butenyl.

The term "alkynyl" signifies a straight-chain or branched alkynyl group with one triple bond and 2 to 8 carbon atoms, preferably 2 to 6 carbon atoms, e.g. propynyl and butynyl.

The term "cycloalkyl" signifies a 4- to 7-membered alicyclic group, namely cyclobutyl, cyclopentyl, cyclohexyl or cycloheptyl, of which cyclopentyl and cyclohexyl are preferred.

The term "unsubstituted or substituted aryl" or equally "aryl which may be substituted" or "optionally mono- or multiply-substituted aryl" preferably embraces a phenyl or naphthyl group, which can be unsubstituted, mono-substituted or multiply-substituted. As substitutents come into consideration e.g. phenyl, halogen and straight-chain and branched alkyl and alkoxy groups with in each case 1 to 5 carbon atoms, whereby the multiply-substituted phenyl or naphthyl groups can have the same or different substituents. Of the alkyl and alkoxy groups the methyl and, respectively, methoxy group is preferred. Examples of optionally substituted aryl groups are phenyl, chloro-, bromo- and fluorophenyl, tolyl, anisyl, as well as naphthyl. The place of the substitution can be at any position of the aromatic ring.

The term "heteroaryl" embraces 5- or 6-membered heterocyclic groups featuring O, S or N as a ring member, i.e. heteroatom, such as, for example, furyl, thienyl, benzofuryl, dibenzofuryl, xanthenyl, pyrrolyl and pyridinyl. The heterocyclic groups featuring O as the heteroatom are especially preferred.

Therefore a further embodiment of the present invention is a transfer hydrogenation (TH⁹), which is (TH), (TH¹), or (TH⁴), wherein
wherein the catalyst of formula (III)
- M: is chosen from the group consisting of Ir, Rh and Ru, preferably Ru, and
- X: signifies H or Cl, and
- Y: is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
- Z: signifies a group of formula (IV) 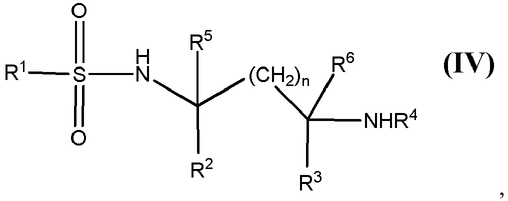
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3.

Furthermore the present invention also relates to preferred transfer hydrogenations wherein the catalyst is of formula (III') 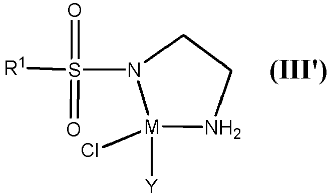 wherein
M is Ru, Rh, Ir, preferably Ru, and
Y is an aromatic ligand chosen from the group consisting of 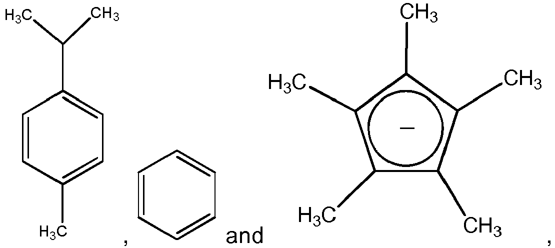 and
R¹ is -CH₃,

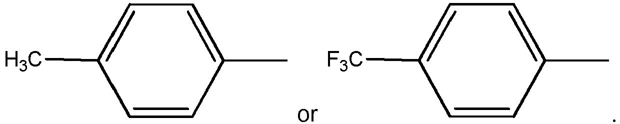

Therefore a further embodiment of the present invention is a transfer hydrogenation (TH¹¹), which is (TH), (TH¹), (TH⁴), or (TH⁹), wherein
the catalyst is of formula (III') 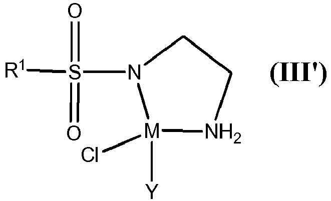 wherein
M is Ru, Rh, Ir, preferably Ru, and
Y is an aromatic ligand chosen from the group consisting of 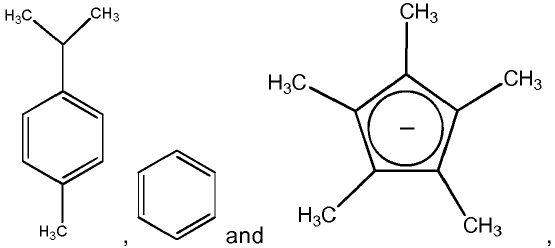 and
R¹ is -CH₃,

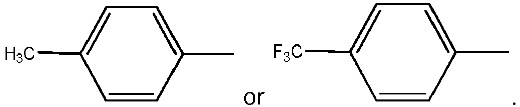

Preferred transfer hydrogenations according to the present invention are such wherein the following catalysts are used: 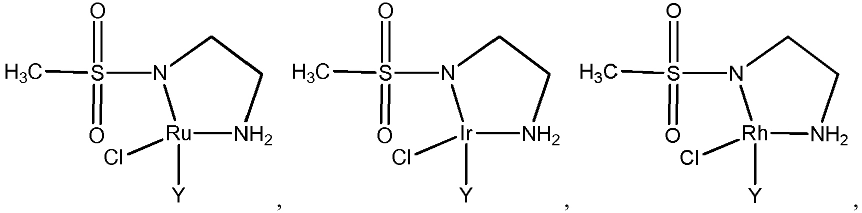 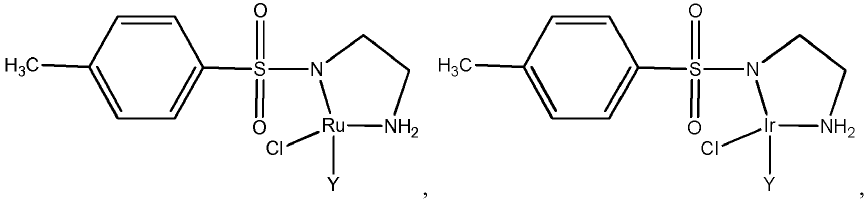 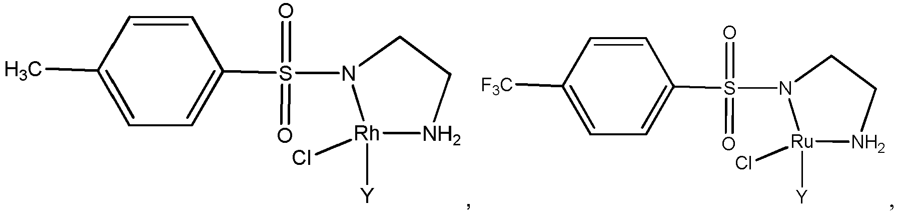 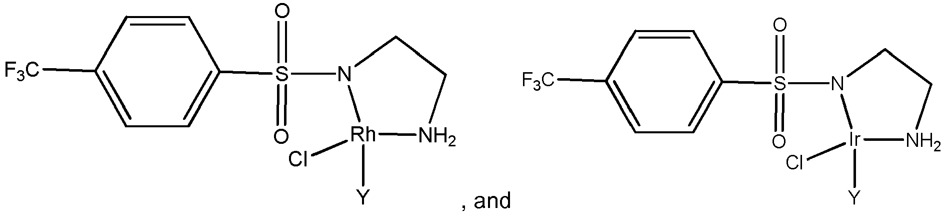 wherein Y is an aromatic ligand chosen from the group consisting of

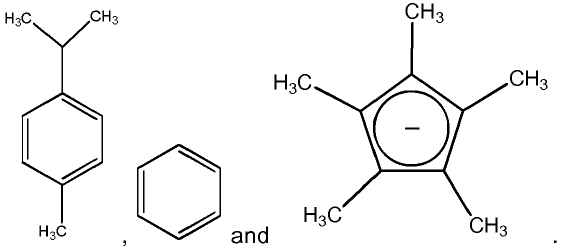

Therefore a further embodiment of the present invention is a transfer hydrogenation (TH¹²), which is (TH), (TH¹), (TH⁴), (TH⁹), or (TH¹¹), wherein the catalyst is chosen from the group consisting of 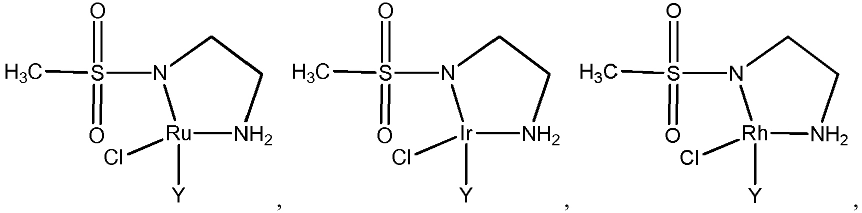 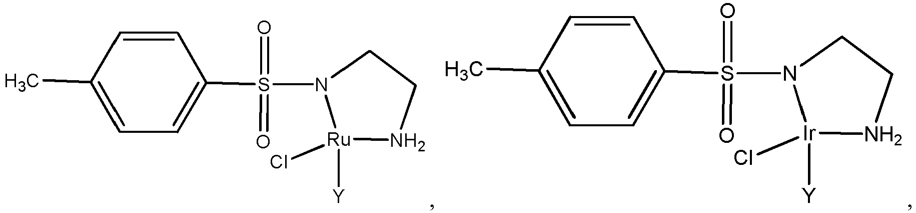 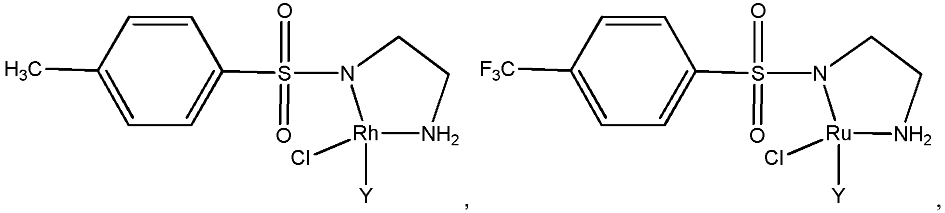 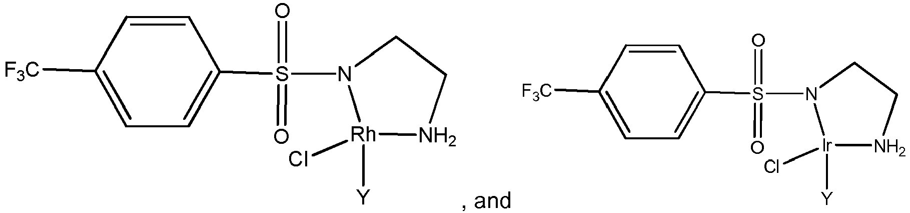 wherein Y is an aromatic ligand chosen from the group consisting of

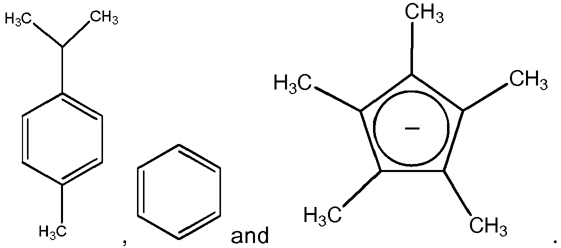

More preferred transfer hydrogenations according to the present invention are such wherein the following catalysts are used: 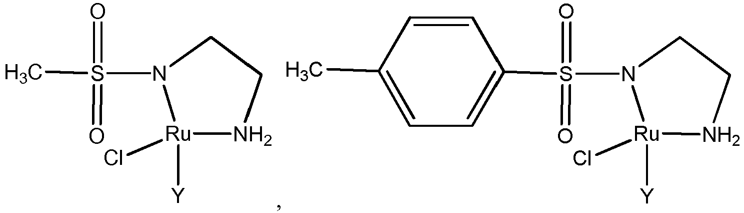 and 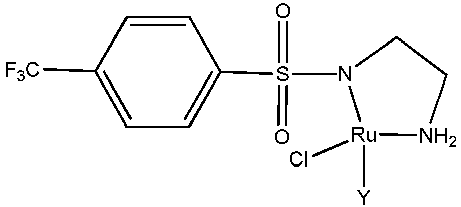 wherein Y is an aromatic ligand chosen from the group consisting of

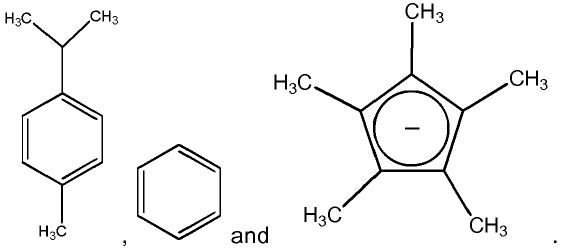

Therefore a further embodiment of the present invention is a transfer hydrogenation (TH¹³), which is (TH), (TH¹), (TH⁴), (TH⁹), (TH¹¹) or (TH¹²), wherein the catalyst is chosen from the group consisting of 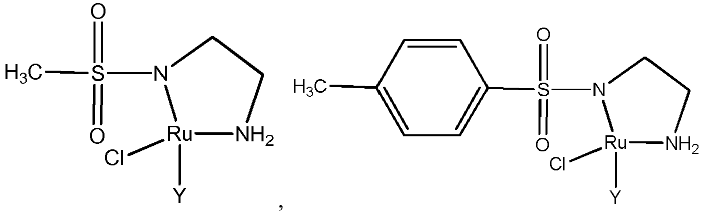 and 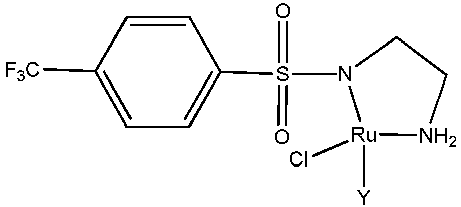 wherein Y is an aromatic ligand chosen from the group consisting of

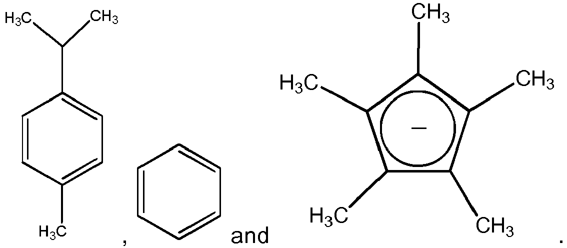

Preferably the catalyst is prepared in situ starting from a metal precursor complex and various ligands:
0.5 Mol [M(Y)X₂]₂ and 1 Mol Z, which is as stated above represented by formula (IV) or formula (V) 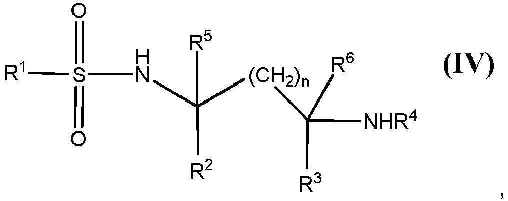 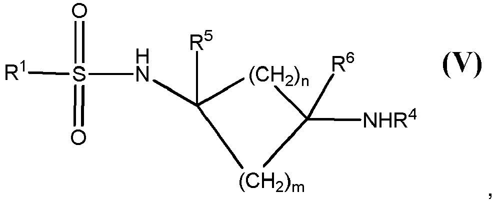 are mixed to form the catalyst.
All the substituents (in formula (IV) and in formula (V)) have the same meanings as defined above as well as the same preferences.

The reaction temperature of the process of production of the catalyst (compound of formula (III)) is usually 0 - 100°C. The reactants are usually mixed for a period of time and then all the reactants for the selective transfer hydrogenation of the present invention are added and the necessary reaction conditions of the hydrogenation are applied.

The selective transfer hydrogenation reaction is usually carried out at a temperature of 0 - 100 °C, preferably 25 - 90°C.

Therefore a further embodiment of the present invention is a transfer hydrogenation (TH¹⁴), which is (TH), (TH¹), (TH⁴), (TH⁹), (TH¹¹), (TH¹²) or (TH¹³), wherein the transfer hydrogenation reaction is carried out at a temperature of 0 - 100 °C, preferably 25 - 90 °C.

The selective transfer hydrogenation reaction is usually carried out at a normal (ambient) pressure. It could also been carried out at elevated and preferably at reduced pressure. Usually the transfer hydrogenation according to the present invention is carried out at a pressure of 50 - 1000 mbar.

Therefore a further embodiment of the present invention is a transfer hydrogenation (TH¹⁵), which is (TH), (TH¹), (TH⁴), (TH⁹), (TH¹¹), (TH¹²), (TH¹³) or (TH¹⁴), wherein the transfer hydrogenation reaction is carried out at a pressure of 50 - 1000 mbar.

The substrate to catalyst ratio (s/c ratio) in the selective transfer hydrogenation is usually from 20:1 to 10000:1, preferably 50:1 to 1000:1. This ratio is related to mol. Mol of substrate (which is compound of formula (I)) to mol of catalyst (compound of formula (III)).

Therefore a further embodiment of the present invention is a transfer hydrogenation (TH¹⁶), which is (TH), (TH¹), (TH⁴), (TH⁹), (TH¹¹), (TH¹²), (TH¹³), (TH¹⁴) or (TH¹⁵), wherein molar substrate to catalyst ratio (s/c ratio) is from 20:1 to 10000:1, preferably from 50:1 to 1000:1.

The transfer hydrogenation according to the present invention allows obtaining retinol in excellent yield.
The retinol which is obtained by the transfer hydrogenation according to the present invention can be used as such or in further application as well as a starting material for further synthesis.

The invention is illustrated by the following Examples. All temperatures are given in °C and all parts and percentages are related to the weight.

### Examples

### Example 1: Production of the catalysts

In a 25 mL 2-necked round bottomed flask equipped with thermometer and a magnetic stirrer, 0.020 mmol of metal complex (M(Y)X₂]₂) and 0.044 mmol of a ligand (Z) were dissolved in 9 mL solvent at room temperature. The mixture was stirred for 30 min.

The solvent was ethyl acetate, isopropanol or methanol.

All of the following catalysts [(C1) - (C8)] were produced according to the process of Example 1
*Catalyst 1 (C1)* 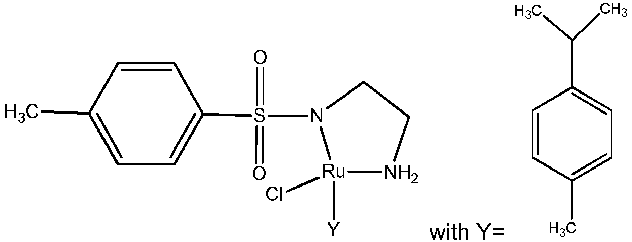
*Catalyst 2 (C2)* 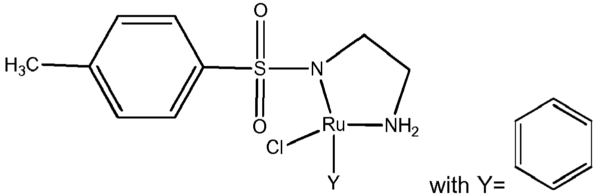
*Catalyst 3 (C3)* 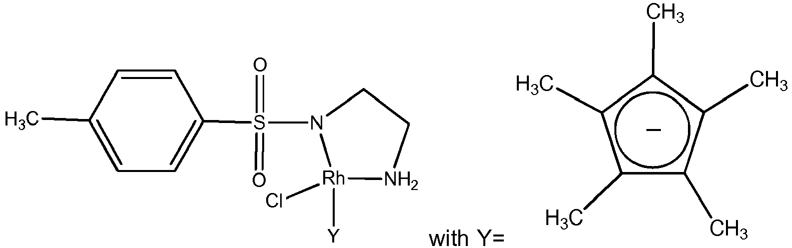
*Catalyst 4 (C4)* 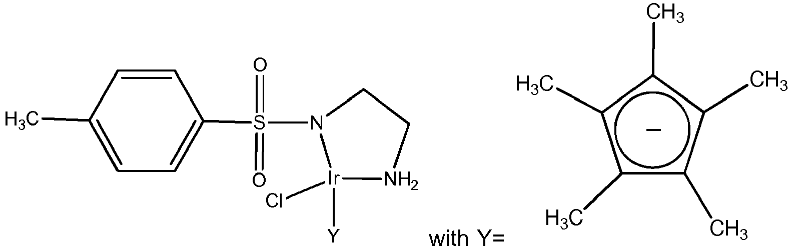
*Catalyst 5 (C5)* 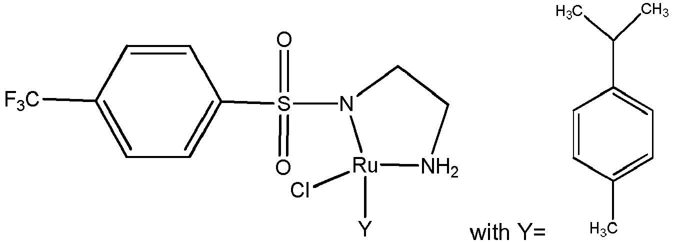
*Catalyst 6 (C6)* 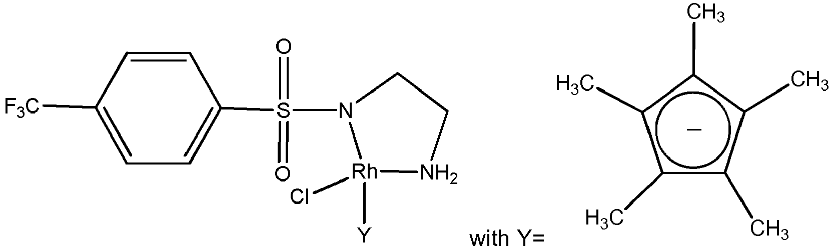
*Catalyst 7 (C7)* 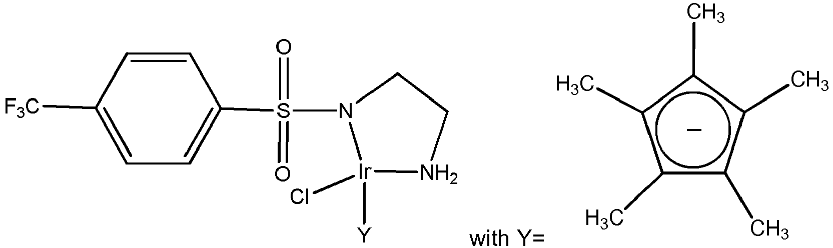
*Catalyst 8 (C8)*

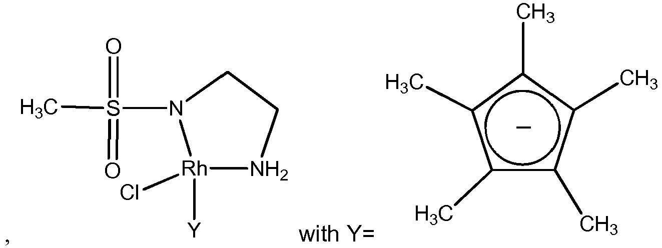

### Example 3a and 3b (Procedure B):

### Preparation of the active catalyst solution

In a 5 ml glass vial under argon atmosphere a stock solution of the catalyst was prepared as follows: 41.4 mg of catalyst C1 were weighed into a glass vial with magnetic stir bar. The vial was capped with a septum and flushed with argon. Then, 900 µl of dichloromethane and a solution of 41.1 mg of sodium formate in 465 µl of deionized water were added. The biphasic mixture was warmed to 35 °C in a water-bath and stirred for 30 min. After phase separation, samples of the organic phase were used as active catalyst solution for several reactions on the same day.

### Reaction

0.73 g of retinal were weighed into a 20 ml glass vial equipped with magnetic stir bar. The vial was sealed with a septum and flushed with argon via cannula. 4.29 ml of anhydrous isopropanol were added via syringe and the mixture was warmed to 38 °C in a water-bath until all retinal had dissolved. After that, 263 µl (1.0 mol%) of the freshly prepared catalyst solution were added via microliter-syringe at 38 °C and the reaction was monitored by TLC and HPLC. After 30 min the reaction was complete. For results see Table.

**Table: with Cat C1 and i-propanol/ sodium formate as red agent and i-propanol as solvent**

| **Example** | **Conc. [ml/mmol]** | **s/c** | **T [°C]** | **t [h]** | **Procedure** | **Conv. [%]** | **Yield [%]** |
|---|---|---|---|---|---|---|---|
| 3a | 3.25 | 111 | 38 | 1 | B | >99.9 | 99.6 |
| 3b | 1.72 | 100 | 38 | 3 | B | 99.2 | 88.4 |

## Claims

1. Transfer hydrogenation of a compound of formula (I) 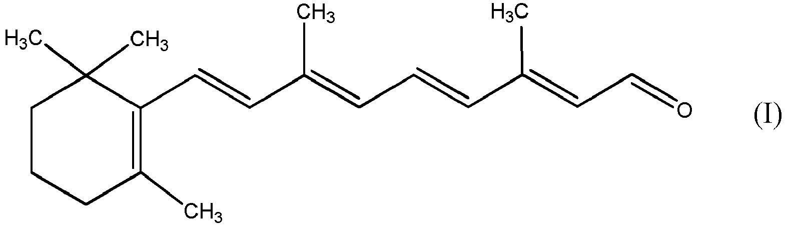 to a compound of formula (II) 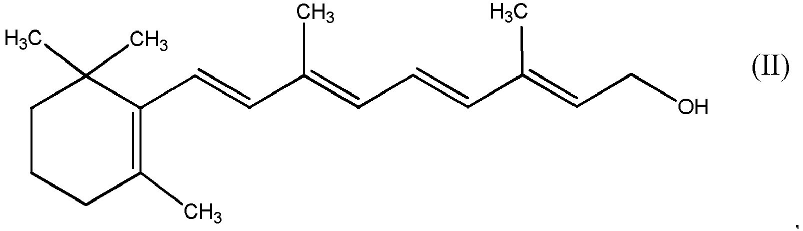 **characterized in that** the hydrogenation is carried out in the presence of
a) an amino-amide-transition-metal catalyst of formula (III)
MX[Z-(-H)][Y] (III)
wherein
M is chosen from the group consisting of Ir, Rh and Ru, and
X signifies H or a halogen atom, and
Y is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
Z signifies a group of formula (IV) or (V) 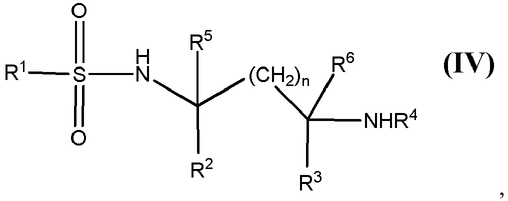 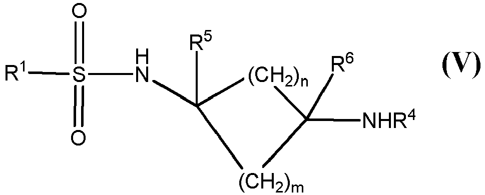
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3, and
m signifies 0,1,2 or 3, and
with the proviso that in formula (V) the sum of n and m is 3 or 4, and
b) a H₂ donor, and wherein the hydrogenation is carried out by using at least one solvent which also serves as hydrogen donor.

2. Transfer hydrogenation according to claim 1, wherein the hydrogenation is carried out in the absence of water.

3. Transfer hydrogenation according to claims 1 or 2, wherein the hydrogenation is carried out by using isopropanol as hydrogen donor and solvent.

4. Transfer hydrogenation according to anyone of the preceding claims, wherein the catalyst of formula (III)
M is chosen from the group consisting of Ir, Rh and Ru, and
X signifies H or Cl, and
Y is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
Z signifies a group of formula (IV) 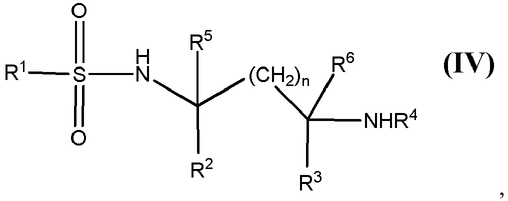
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3.

5. Transfer hydrogenation according to anyone of the preceding claims, wherein the catalyst is of formula (III') 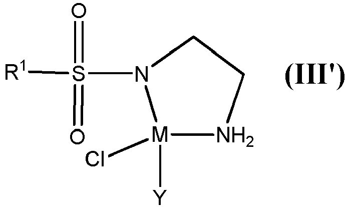 wherein
M is Ru, Rh or Ir, and
Y is an aromatic ligand chosen from the group consisting of 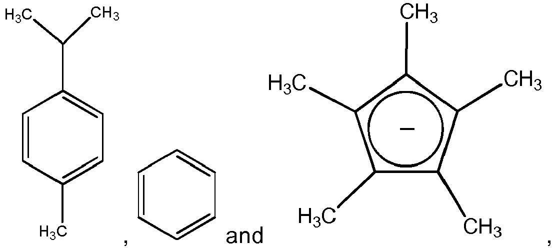 and
R¹ is -CH₃,

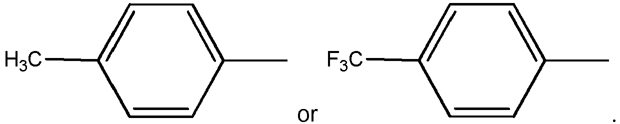

6. Transfer hydrogenation according to anyone of the preceding claims, wherein the catalyst is chosen from the group consisting of 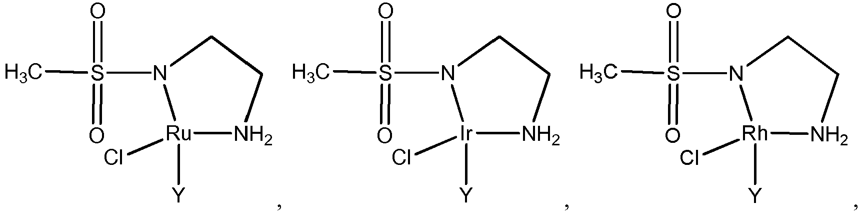 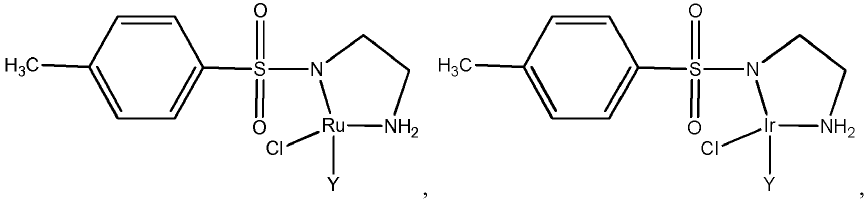 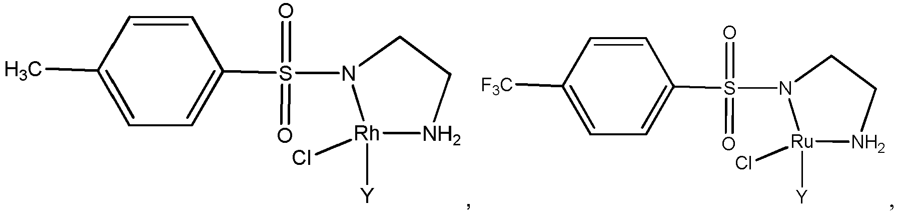 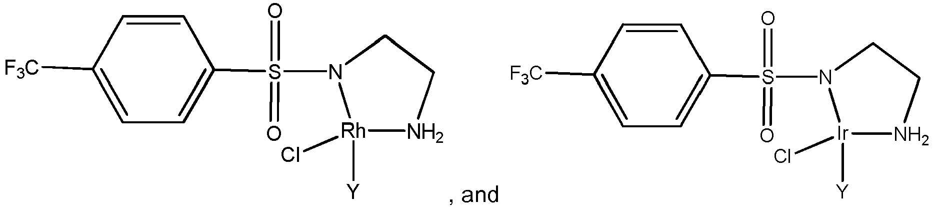 wherein Y is an aromatic ligand chosen from the group consisting of

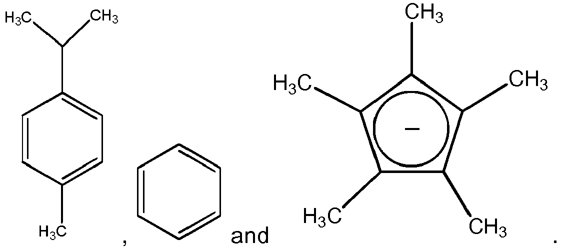

7. Transfer hydrogenation according to anyone of the preceding claims 1 - 5, wherein the catalyst is chosen from the group consisting of 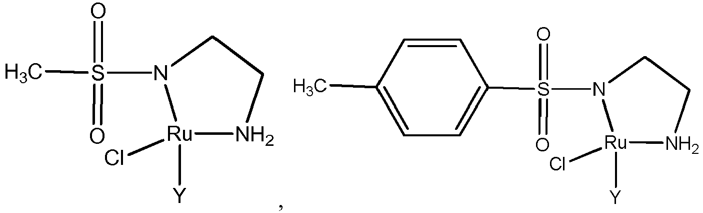 and 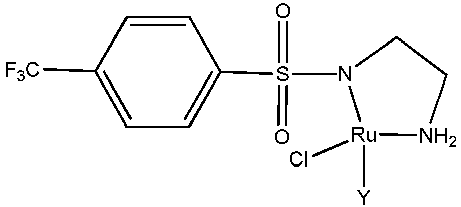 wherein Y is an aromatic ligand chosen from the group consisting of

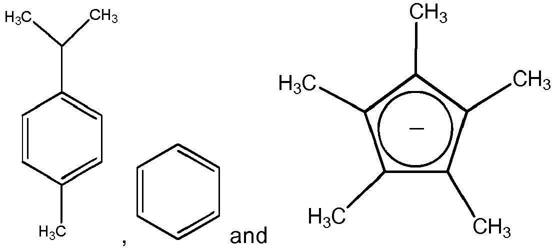

8. Transfer hydrogenation according to anyone of the preceding claims, wherein transfer hydrogenation reaction is carried out at a temperature of 0 - 100 °C.

9. Transfer hydrogenation according to anyone of the preceding claims, wherein the transfer hydrogenation reaction is carried out at a pressure of 50 - 1000 mbar.

10. Transfer hydrogenation according to anyone of the preceding claims, wherein the molar substrate to catalyst ratio (s/c ratio) is from 20:1 to 10000:1.

## Patentansprüche

1. Transferhydrierung einer Verbindung der Formel (I) (I) 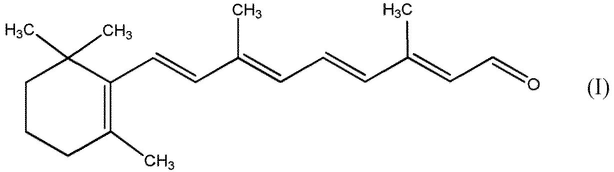 zu einer Verbindung der Formel (II), 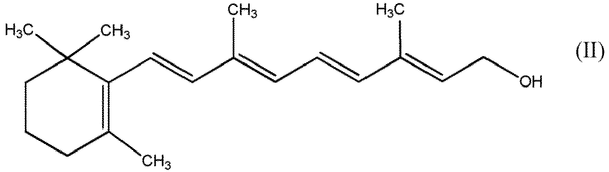 **dadurch gekennzeichnet, dass** die Hydrierung in Gegenwart von
a) einem Amino-Amid-Übergangsmetallkatalysator der Formel (III),
MX[Z-(-H)][Y] (III)
wobei
M ausgewählt ist aus der Gruppe bestehend aus Ir, Rh und Ru und
X H oder ein Halogenatom bedeutet und
Y ausgewählt ist aus der Gruppe bestehend aus Pentamethylcyclopentadienyl, Benzol, p-Cymol, Toluol, Anisol, Xylol, 1,3,5-Trimethylbenzol, p-Dicyclohexylbenzol, Naphthalin und Tetralin und
Z eine Gruppe der Formel (IV) oder (V) bedeutet, 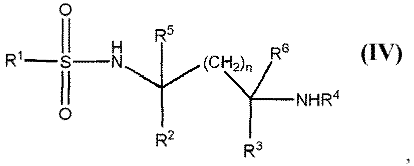 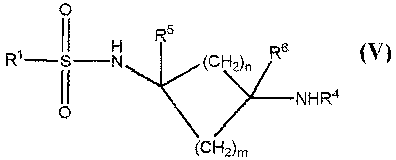
wobei
R¹ C₁-C₂-Alkyl, das mit einem oder mehreren Fluoratomen substituiert sein kann, C₂-C₆-Alkenyl, C₂-C₆-Alkinyl, C₄-C₇-Cycloalkyl, Aryl, das substituiert sein kann, Heteroaryl oder Campher-10-yl bedeutet und
R² und R³ jeweils unabhängig Wasserstoff, C₁-C₂-Alkyl, C₄-C₇-Cycloalkyl oder Aryl, das substituiert sein kann, bedeuten und
R⁴ Wasserstoff oder C₁-C₂-Alkyl bedeutet und
R⁵ Wasserstoff oder C₁-C₂-Alkyl bedeutet und
R⁶ Wasserstoff oder C₁-C₂-Alkyl bedeutet und
n 0, 1, 2 oder 3 bedeutet und
m 0, 1, 2 oder 3 bedeutet und
mit der Maßgabe, dass in Formel (V) die Summe von n und m 3 oder 4 beträgt, und
b) einem H₂-Donator
durchgeführt wird, und wobei die Hydrierung unter Verwendung wenigstens eines Lösungsmittels, das auch als Wasserstoffdonator wirkt, durchgeführt wird.

2. Transferhydrierung gemäß Anspruch 1, wobei die Hydrierung ohne Vorhandensein von Wasser durchgeführt wird.

3. Transferhydrierung gemäß Anspruch 1 oder 2, wobei die Hydrierung unter Verwendung von Isopropanol als Wasserstoffdonator und Lösungsmittel durchgeführt wird.

4. Transferhydrierung gemäß einem der vorstehenden Ansprüche, wobei bei dem Katalysator der Formel (III)
M ausgewählt ist aus der Gruppe bestehend aus Ir, Rh und Ru und
X H oder Cl bedeutet und
Y ausgewählt ist aus der Gruppe bestehend aus Pentamethylcyclopentadienyl, Benzol, p-Cymol, Toluol, Anisol, Xylol, 1,3,5-Trimethylbenzol, p-Dicyclohexylbenzol, Naphthalin und Tetralin und
Z eine Gruppe der Formel (IV) bedeutet, 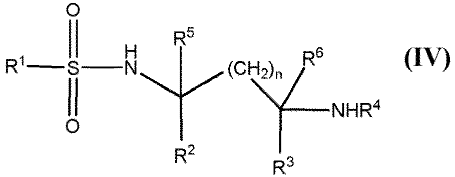
wobei
R¹ C₁-C₂-Alkyl, das mit einem oder mehreren Fluoratomen substituiert sein kann, C₂-C₆-Alkenyl, C₂-C₆-Alkinyl, C₄-C₇-Cycloalkyl, Aryl, das substituiert sein kann, Heteroaryl oder Campher-10-yl bedeutet und
R² und R³ jeweils unabhängig Wasserstoff, C₁-C₂-Alkyl, C₄-C₇-Cycloalkyl oder Aryl, das substituiert sein kann, bedeuten und
R⁴ Wasserstoff oder C₁-C₂-Alkyl bedeutet und
R⁵ Wasserstoff oder C₁-C₂-Alkyl bedeutet und
R⁶ Wasserstoff oder C₁-C₂-Alkyl bedeutet und
n 0, 1, 2 oder 3 bedeutet.

5. Transferhydrierung gemäß einem der vorstehenden Ansprüche, wobei der Katalysator von Formel (III') ist, 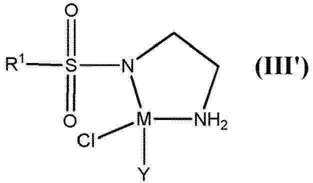 wobei
M Ru, Rh oder Ir ist und
Y ein aromatischer Ligand ausgewählt aus der Gruppe bestehend aus 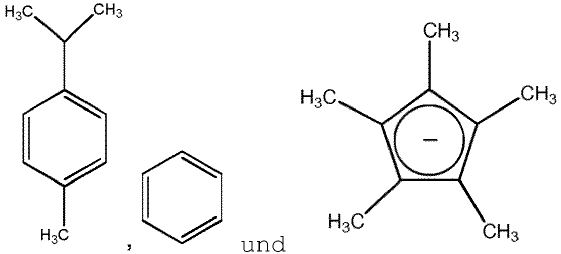 ist und
R₁ -CH₃, 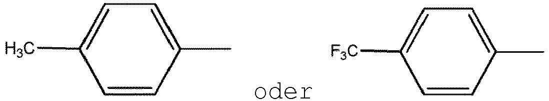 ist.

6. Transferhydrierung gemäß einem der vorstehenden Ansprüche, wobei der Katalysator ausgewählt ist aus der Gruppe bestehend aus 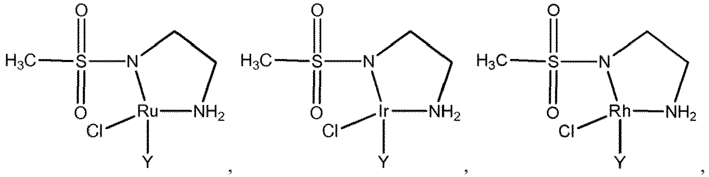 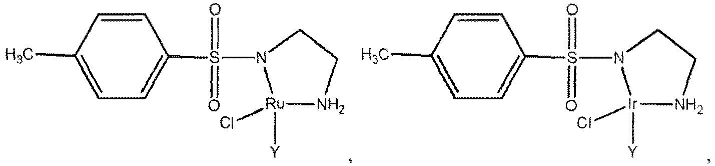 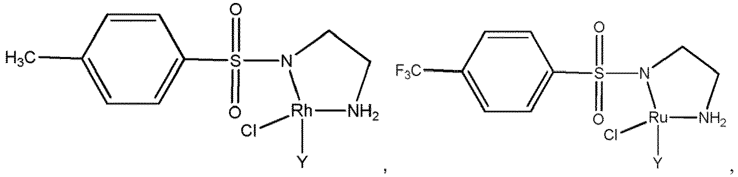 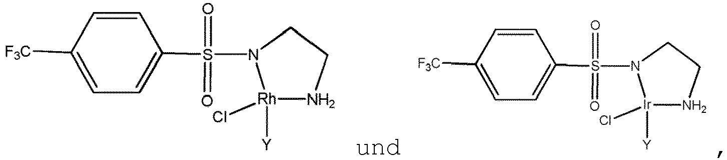 wobei Y ein aromatischer Ligand ausgewählt aus der Gruppe bestehend aus 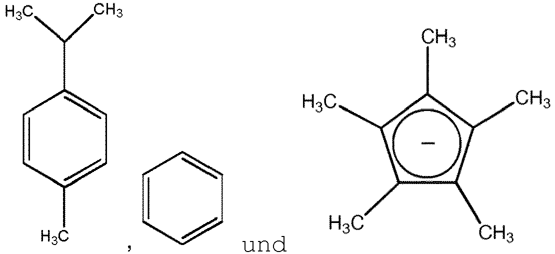 ist.

7. Transferhydrierung gemäß einem der vorstehenden Ansprüche 1-5, wobei der Katalysator ausgewählt ist aus der Gruppe bestehend aus 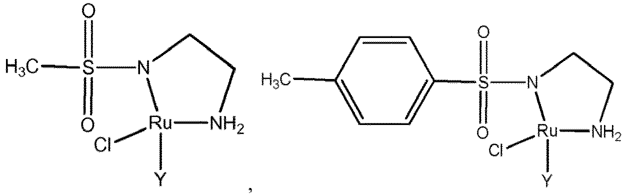 und 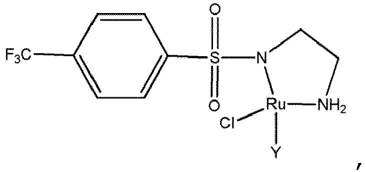 wobei Y ein aromatischer Ligand ausgewählt aus der Gruppe bestehend aus 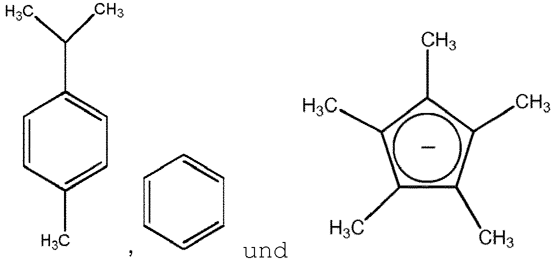 ist.

8. Transferhydrierung gemäß einem der vorstehenden Ansprüche, wobei die Transferhydrierungsreaktion bei einer Temperatur von 0-100 °C durchgeführt wird.

9. Transferhydrierung gemäß einem der vorstehenden Ansprüche, wobei die Transferhydrierungsreaktion bei einem Druck von 50-1000 mbar durchgeführt wird.

10. Transferhydrierung gemäß einem der vorstehenden Ansprüche, wobei das Molverhältnis von Substrat zu Katalysator (s/c-Verhältnis) von 20:1 bis 10000:1 beträgt.

## Revendications

1. Hydrogénation par transfert d'un composé de formule (I) 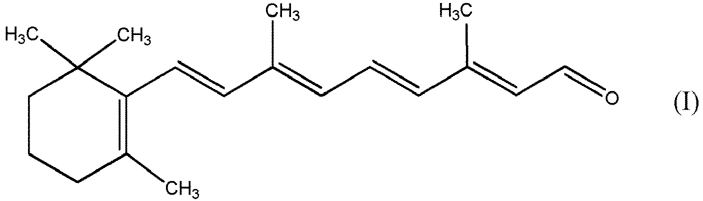 en un composé de formule (II) 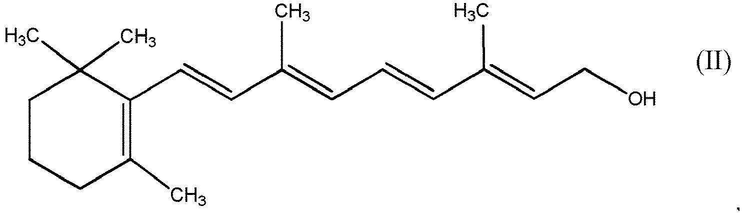 **caractérisée en ce que** l'hydrogénation est effectuée en présence de
a) un catalyseur amino-amide-métal de transition de formule (III)
MX[Z-(-H)][Y] (III)
dans lequel
M est choisi dans le groupe constitué par Ir, Rh et Ru et
X désigne H ou un atome d'halogène et
Y est choisi dans le groupe constitué par le groupe pentaméthylcyclopentadiényle, le benzène, le *p*-cymène, le toluène, l'anisole, le xylène, le 1,3,5-triméthylbenzène, le *p-*dicyclohexylbenzène, le naphtalène et la tétraline et
Z désigne un groupe de formule (IV) ou (V) 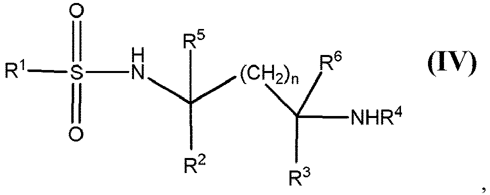 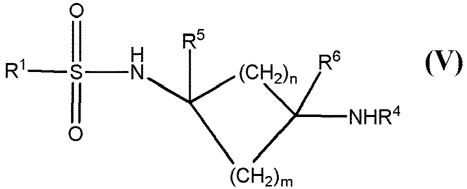
dans lequel
R¹ désigne un groupe alkyle en C₁-C₂ qui peut être substitué par un ou plusieurs atomes de fluor, alcényle en C₂-C₆, alcynyle en C₂-C₆, cycloalkyle en C₄-C₇, aryle qui peut être substitué, hétéroaryle ou camphor-10-yle et
R² et R³ désignent chacun indépendamment l'atome d'hydrogène ou un groupe alkyle en C₁-C₂, cycloalkyle en C₄-C₇ ou aryle qui peut être substitué et
R⁴ désigne l'atome d'hydrogène ou un groupe alkyle en C₁-C₂ et
R⁵ désigne l'atome d'hydrogène ou un groupe alkyle en C₁-C₂ et
R⁶ désigne l'atome d'hydrogène ou un groupe alkyle en C₁-C₂ et
n désigne 0, 1, 2 ou 3 et
m désigne 0, 1, 2 ou 3 et
à condition que dans la formule (V) la somme de n et m soit de 3 ou 4, et
b) un donneur de H₂,
et l'hydrogénation étant effectuée à l'aide d'au moins un solvant qui sert également de donneur d'hydrogène.

2. Hydrogénation par transfert selon la revendication 1, l'hydrogénation étant effectuée en l'absence d'eau.

3. Hydrogénation par transfert selon les revendications 1 ou 2, l'hydrogénation étant effectuée à l'aide d'isopropanol en tant que donneur d'hydrogène et solvant.

4. Hydrogénation par transfert selon l'une quelconque des revendications précédentes, dans laquelle dans le catalyseur de formule (III)
M est choisi dans le groupe constitué par Ir, Rh et Ru et
X désigne H ou Cl et
Y est choisi dans le groupe constitué par le groupe pentaméthylcyclopentadiényle, le benzène, le *p-*cymène, le toluène, l'anisole, le xylène, le 1,3,5-triméthylbenzène, le p-dicyclohexylbenzène, le naphtalène et la tétraline et
Z désigne un groupe de formule (IV) 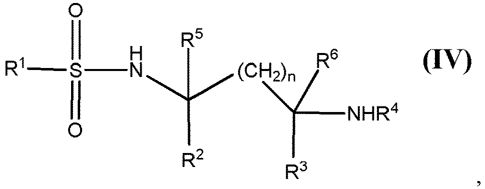
dans lequel
R¹ désigne un groupe alkyle en C₁-C₂ qui peut être substitué par un ou plusieurs atomes de fluor, alcényle en C₂-C₆, alcynyle en C₂-C₆, cycloalkyle en C₄-C₇, aryle qui peut être substitué, hétéroaryle ou camphor-10-yle et
R² et R³ désignent chacun indépendamment l'atome d'hydrogène ou un groupe alkyle en C₁-C₂, cycloalkyle en C₄-C₇ ou aryle qui peut être substitué et
R⁴ désigne l'atome d'hydrogène ou un groupe alkyle en C₁-C₂ et
R⁵ désigne l'atome d'hydrogène ou un groupe alkyle en C₁-C₂ et
R⁶ désigne l'atome d'hydrogène ou un groupe alkyle en C₁-C₂ et
n désigne 0, 1, 2 ou 3.

5. Hydrogénation par transfert selon l'une quelconque des revendications précédentes, dans laquelle le catalyseur est de formule (III') 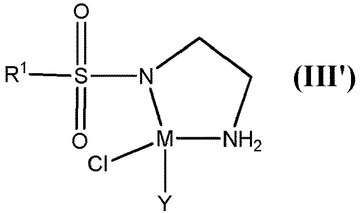 dans laquelle
M est Ru, Rh ou Ir et
Y est un ligand aromatique choisi dans le groupe constitué par 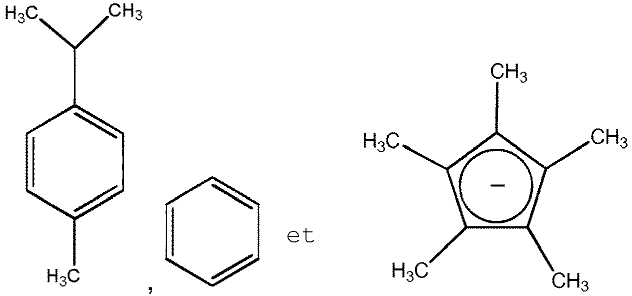 et
R¹ est -CH₃,

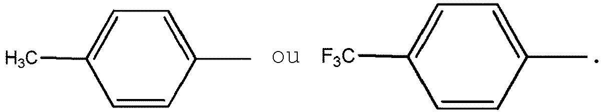

6. Hydrogénation par transfert selon l'une quelconque des revendications précédentes, dans laquelle le catalyseur est choisi dans le groupe constitué par 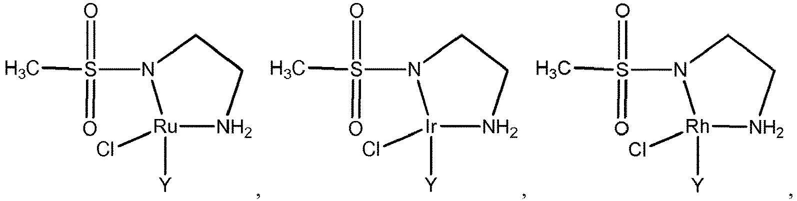 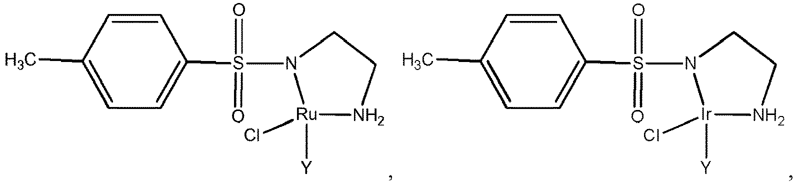 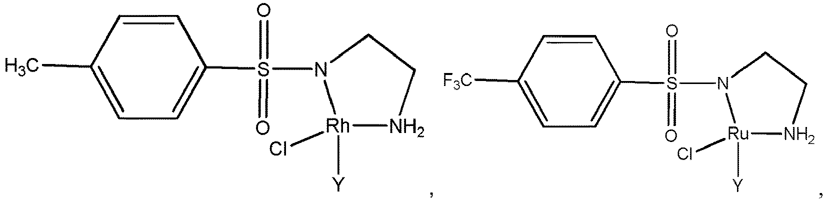 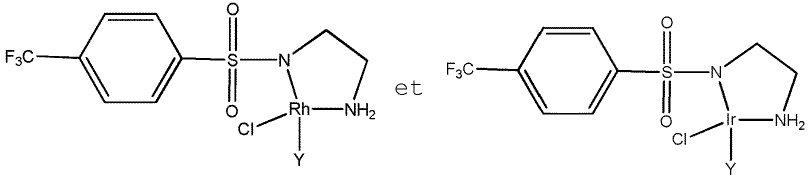 dans lesquels Y est un ligand aromatique choisi dans le groupe constitué par

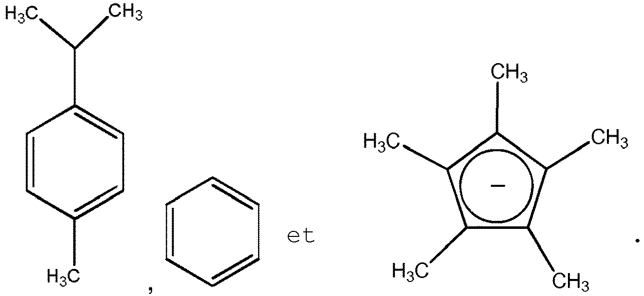

7. Hydrogénation par transfert selon l'une quelconque des revendications 1-5 précédentes, dans laquelle le catalyseur est choisi dans le groupe constitué par 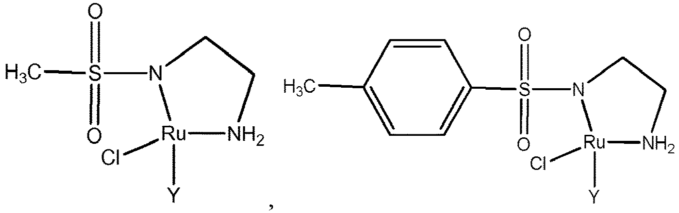 et 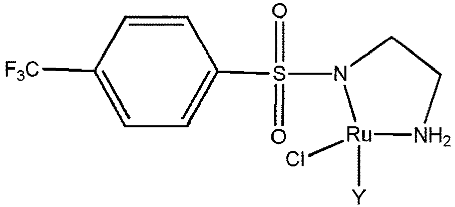 dans lesquels Y est un ligand aromatique choisi dans le groupe constitué par

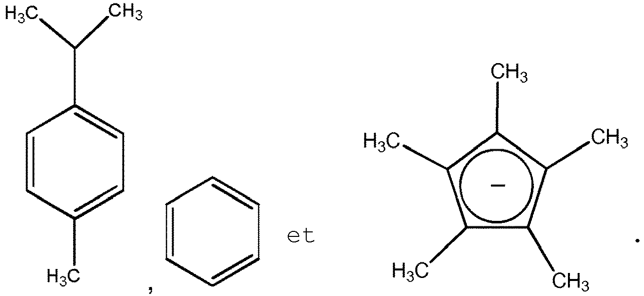

8. Hydrogénation par transfert selon l'une quelconque des revendications précédentes, dans laquelle la réaction d'hydrogénation par transfert est effectuée à une température de 0-100 °C.

9. Hydrogénation par transfert selon l'une quelconque des revendications précédentes, dans laquelle la réaction d'hydrogénation par transfert est effectuée à une pression de 50-1000 mbar.

10. Hydrogénation par transfert selon l'une quelconque des revendications précédentes, dans laquelle le rapport molaire du substrat au catalyseur (rapport s/c) est de 20:1 à 10000:1.
